# EUROPÄISCHE PATENTSCHRIFT

(11) **EP 2 210 028 B1**
(45) Veröffentlichungstag und Bekanntmachung des Hinweises auf die Patenterteilung: **07.12.2011**
(21) Anmeldenummer: 08839219.6
(22) Anmeldetag: 10.10.2008
(51) Int. Cl.: F16L 3/223

(54) **HALTERUNG FÜR HEIZUNGSROHRE**
HOLDER FOR HEATING PIPES
SUPPORT POUR TUBES CHAUFFANTS

(30) Priorität: 10.10.2007 DE 202007014168 U
(43) Veröffentlichungstag der Anmeldung: 28.07.2010
(73) Patentinhaber: Rehau AG + Co, 95111 Rehau (DE)
(72) Erfinder: LIEBEL, Volker, 91058 Erlangen (DE)
(86) Internationale Anmeldenummer: PCT/EP2008/008567
(87) Internationale Veröffentlichungsnummer: WO 2009/049828

(56) Entgegenhaltungen:
- EP-A2- 0 860 672
- EP-A2- 0 866 281
- AT-B- 408 804
- DE-A1- 2 300 800
- DE-A1- 2 300 800
- DE-A1- 3 219 464
- DE-A1- 3 219 464
- DE-U1- 29 908 173
- DE-U1- 29 908 173
- US-A- 2 362 124
- US-A- 2 362 124

## Beschreibung

Die Erfindung betrifft eine Halterung für Heizungsrohre in Anlagen zur Biogaserzeugung und eine Vorrichtung zur Heizung.

In Anlagen zur Biogaserzeugung muss die Biomasse auf eine Temperatur von typischerweise 40 bis 55°C erwärmt werden, um eine effiziente Biogasproduktion zu gewährleisten.

Nach dem heutigen Stand der Technik werden dazu entweder Rohre aus Edelstahl oder aus polymeren Werkstoffen, bevorzugt aus vernetztem Polyethylen (PE-Xa) verwendet, die vor der Biomasse-Behälterwand angeordnet sind.

Die Halterung der Rohre aus Edelstahl muss, um die Ausdehnungskräfte aufnehmen zu können, ebenfalls aus Edelstahl sein. Damit ist es einfach möglich, die Halterung der Rohre so auszuführen, dass die Heizungsrohre einen beliebigen Abstand von der Behälterwand aufweisen, um einen effektiven Wärmeeintrag in die Biomasse zu gewährleisten und um zu verhindern, dass sich zwischen den Heizungsrohren und der Behälterwand Biomasse ansammelt und festsetzt. Nachteilig bei dieser Edelstahlausführung sind die hohen Material- und Montagekosten sowie - auch bei besten Stahlqualitäten - Korrosion insbesondere an den Schweißnähten. Beispielsweise aus der EP 0 866 281 A2, der EP 0 860 672 A2 oder der AT 408 804 B sind bekannte Beispiele zur Halterung bzw. Beabstandung von Heizungsrohren aus anderen, entfernt liegenden technischen Gebieten bekannt. Weitere bekannte Beispiele finden sich in der DE 2 300 800 A1, der DE 32 19 464 A1 und der DE 299 08 173 U1.

Der Nachteil polymerer Heizungsrohre besteht darin, dass mit den bisher bekannten Halterungssystemen der für einen dauerhaft effektiven und störungsfreien Heizbetrieb erforderliche Abstand der Heizungsrohre von der Behälterwand nicht gewährleistet werden kann. Verwendet werden bisher Schienen, in die die Heizungsrohre einrastend befestigt werden. Der damit erreichbare Abstand der Rohre von der Behälterwand beträgt nur wenige Zentimeter, wodurch ein dauerhaft störungsfreier Betrieb nicht gewährleistet werden kann. Bei weiteren bekannten Ausführungsformen werden diese Schienen mit vergrößerter Tiefe ausgeführt oder zur Beabstandung von der Behälterwand auf einer zusätzlichen durchgehenden Schiene montiert.

Dabei ist aber der zusätzliche erhebliche Materialbedarf sowie die Tatsache nachteilig, dass damit eine viele Zentimeter in das Behälterinnere hineinragende Wand geschaffen wird, die die zur Durchmischung der Biomasse erforderliche Strömung erheblich behindert. Hier setzt die Erfindung ein, mit der die Nachteile des Standes der Technik überwunden werden sollen.

Die erfindungsgemäße Halterung für Heizungsrohre in Anlagen zur Biogaserzeugung gemäß Anspruch 1 zeichnet sich dadurch aus, dass die Heizungsrohrführung und der Abstandshalter Holz enthalten.

Der Vorteil der Verwendung von Holz besteht darin, dass dieses in der vorgesehenen Anwendung sehr widerstandsfähig gegen das in der Anlage zur Biogaserzeugung herrschende Milieu ist. Darüber hinaus ist Holz bedingt durch seine Elastizität und Zähigkeit vorteilhaft bei der Aufnahme von Kräften beim Betrieb der Anlage.

Die erfindungsgemäße Halterung ist demnach bevorzugt aus polymeren Werkstoffen hergestellt und besteht aus parallel zur Behälterwand angeordneten Elementen zur Führung der Heizungsrohre, Abstandshaltern, die nur punktuell zwischen Behälterwand und den Elementen zur Führung der Heizungsrohre angeordnet sind, und den Elementen zur Befestigung in der Behälterwand. Um die Abstandshalter vom Gewicht der Halterung zu entlasten, kann eine zusätzliche Abstützung auf dem Boden des zu beheizenden Behälters vorgesehen werden.

Damit kann mit geringem Materialaufwand bei gleichzeitiger minimaler Störung der Durchmischungsströmung im Biomassebehälter ein beliebiger Abstand zwischen Behälterwand und Heizungsrohren realisiert werden. Die Anordnung ist durch eine flexible Auslegung ihrer Komponenten in hoher Stabilität ausführbar und widersteht den auftretenden Strömungskräften. Sie ist einfach, schnell und kostengünstig aus wenigen Komponenten aufzubauen. Materialbedingt wird sie von den chemisch aggressiven Stoffen der Biomasse nicht angegriffen und ist dadurch langlebig und wartungsarm.

In der einfachsten Ausführungsform bestehen die Heizungsrohrführungen und Abstandshalter aus gefrästen oder gebohrten Rohren aus Holz enthaltendem Polymermaterial, und die Befestigungselemente aus Edelstahl.
Die Heizungsrohrführungen und / oder Abstandshalter können erfindungsgemäß auch runde, elliptische oder polygonale Querschnitte aufweisen.

Insbesondere die Abstandshalter können jedoch auch als Formteil ausgeführt sein.

Zur sicheren Aufnahme von Querkräften aufgrund der Biomasseströmung können die Abstandshalter auch mehrteilig ausgeführt sein;

Die erfindungsgemäßen Komponenten bestehen vorzugsweise aus polymeren, bevorzugt vernetzten Materialien, die Befestigungselemente bevorzugt aus Edelstahl.

Als Polymermaterial eignet sich auf Grund seiner Widerstandsfähigkeit insbesondere vernetztes Polyethylen (PE-X) und faserverstärkte Materialien, insbesondere glasfaserverstärkte Duromere (GFK).

Gemäß der vorliegenden Erfindung sind die Halterungen mittels Befestigungselementen so an der Behälterwand angeordnet, dass die Abstandshalter zwischen der Heizungsrohrführung und der Behälterwand verspannt sind.

In einer Abwandlung der Erfindung sind die Abstandshalter an der Behälterwand befestigt und die Heizungsrohrführungen mittels separater Befestigungselemente an den Abstandshaltern befestigt.

Die Abstandshalter können als Spritzgussformteil so ausgeführt sein, dass eine formschlüssige Aufnahme der Heizungsrohrführungen angeformt ist.

Besonders vorteilhaft werden als Befestigungselemente Gewindestangen ausgewählt, die in der Behälterwand verankert sind.

Um die Abstandshalter besonders fest an der Behälterwand zu verankern, können die Abstandshalter einen zur Behälterwand hin zunehmenden Querschnitt aufweisen.

Bei Behälterwänden aus Stahl können die Abstandshalter mit diesen auch mittels Verschweißung verbunden sein, bei solchen aus GFK auch mittels Verklebung.

Fixierelemente z.B. in Form von Ringen können die Heizungsrohre gemäß der vorliegenden Erfindung kraftschlüssig in den Heizungsrohrführungen verspannen.

In einer weiteren erfindungsgemäßen Ausführungsform werden die Heizungsrohre in den Heizungsrohrführungen fixiert, indem die Heizungsrohrführungen ausgeschäumt oder mit einem aushärtenden, bevorzugt polymeren Material ausgegossen werden.

Die Heizungsrohrführungen weisen bevorzugt runde Öffnungen zur Aufnahme und Führung der Heizungsrohre auf, um diese formschlüssig zu positionieren.

In einer Abwandlung der vorliegenden Erfindung können die Heizungsrohrführungen Öffnungen aufweisen, in die Heizungsrohre eingelegt oder eingedrückt werden können.
Zur Festlegung der Heizungsrohre ist bevorzugt wenigstens ein Sperrelement vorgesehen.

Die Heizungsrohrführung und/oder der Abstandshalter enthält Holz.
Das Holz kann dabei in verteilter Form als Faser oder Span dem Polymermaterial zugesetzt sein, es kann aber auch vorgesehen sein, die Heizungsrohrführung bzw. den Abstandshalter anteilig bzw. vollständig aus Holz herzustellen.

Die einzelnen Komponenten der erfindungsgemäßen Halterung können auch mehrteilig ausgeführt sein und miteinander insbesondere mittels Verschraubung, Verklebung, Vernietung und Verschweißung verbunden sein.

Eine Vorrichtung zur Heizung insbesondere in Anlagen zur Biogaserzeugung ist mit einer Halterung für Heizungsrohre der dargestellten Art ausgestattet.

Nachfolgend sind beispielhaft, aber die Lehre der Erfindung nicht einschränkend, Ausführungsformen der Erfindung näher erläutert.

Mit Bezug auf die Figuren 1 bis 9 ist dabei folgendes gezeigt:
- Fig.1: stellt eine Seitenansicht einer erfindungsgemäßen Halterung für Heizungsrohre in einer Anlage zur Biogaserzeugung dar;
- Fig. 2: zeigt eine Aufsicht, wobei die Ansicht A nach Fig. 1 wiedergegeben ist;
- Fig. 3: bildet eine weitere Ausführung eines Abstandshalters ab;
- Fig. 4: gibt einen weiteren erfindungsgemäßen Abstandshalter wieder;
- Fig. 5: stellt einen anderen Abstandshalter dar mit einem Rastelement;
- Fig. 6: zeigt einen Abstandshalter in einer abgewandelten Ausführung;
- Fig. 7: gibt ein Fixierelement in Form eines Ringes wieder;
- Fig. 8: stellt eine weitere Ausführungsform einer Halterung für Heizungsrohre in einer Anlage zur Biogaserzeugung in einer Seitenansicht dar;
- Fig. 9: zeigt eine Aufsicht, wobei die Ansicht B nach Fig. 8 wiedergegeben ist;
- Fig. 10: zeigt einen nicht zur Erfindung gehörenden Abstandshalter, der V-förmig ausgeführt ist.

### Erläuterung der Figuren im Detail:

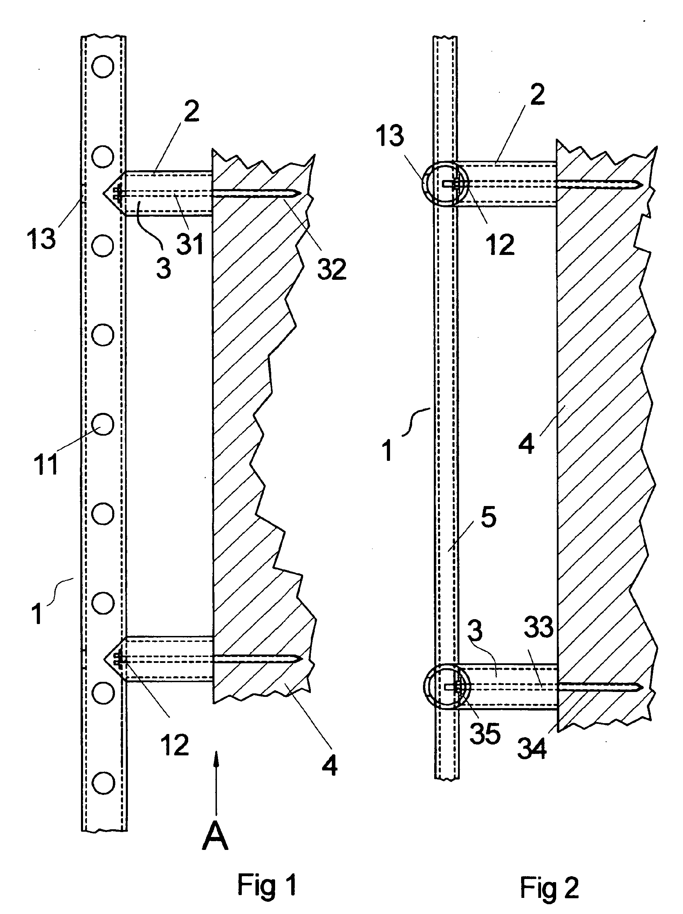

Ab hier bis zum Ende der Beschreibung sind alle Klammerzeichen →"( )" zu entfernen In Fig. 1 und Fig. 2 ist die einfachste Ausführungsform dargestellt, wobei Fig. 2 die Ansicht A der Fig. 1 darstellt. Die hier als Rohre ausgeführte Heizungsrohrführungen (1) und Abstandshalter (2) sind dabei mittels Befestigungselementen (3) parallel bzw. senkrecht zur Behälterwand (4) angeordnet.
Die Heizungsrohrführung (1) weist Öffnungen (11) zur Aufnahme der nur in Fig. 2 dargestellten Heizungsrohre (5) auf. Die Heizungsrohrführung (1) weist weitere Öffnungen (12, 13) auf, die um 90° zu den Öffnungen (11) versetzt sind. Die Öffnung (12) dient der Aufnahme der Befestigungselemente (3), die in Fig. 1 als Schrauben (31) dargestellt sind, die mittels Dübeln (32) in der Behälterwand (4) befestigt sind.
Die Öffnungen (13) dienen der Betätigung der Befestigungselemente (3).

In einer bevorzugten Ausführungsform gemäß Fig. 2 bestehen die Befestigungselemente (3) aus Gewindestangen (33), die in der Behälterwand (4) in einer ausgehärteten Masse (34) befestigt sind. Damit können höhere Auszugskräfte als bei einer Dübelbefestigung und eine sichere Versiegelung des Bohrloches in der Behälterwand gewährleistet werden.

Die dann zur Befestigung der Heizungsrohrführung (1) erforderlichen Muttern (35) werden bevorzugt als Bundmuttern ausgeführt, um auf eine separate Unterlegscheibe verzichten zu können. Vorteilhaft ist in jedem Fall die Ausführung der Bundmutter oder der Unterlegscheiben mit einer Sperrverzahnung, um ein Lösen der Befestigung im Betrieb zu verhindern.

Die erfindungsgemäßen Heizungsrohrführungen (1) und Abstandshalter (2) können abweichend von den in den Darstellungen beispielhaft rund dargestellten Ausführungsformen auch einen elliptischen, polygonalen oder beliebigen anderen Querschnitt aufweisen.

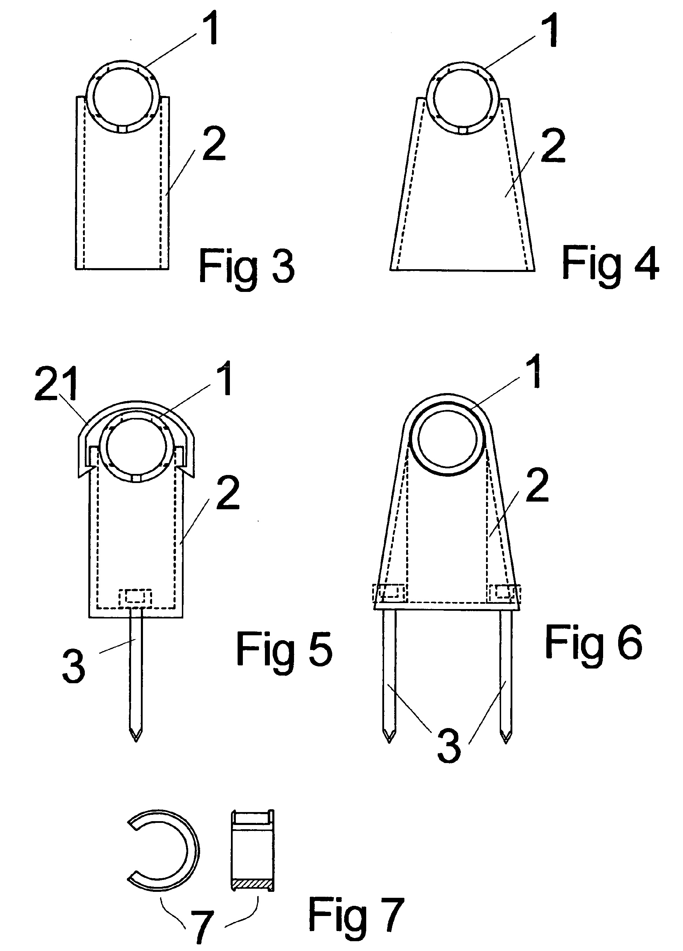

In Fig. 3 ist eine Ausführung der Erfindung dargestellt, bei der die Abstandshalter (2) einen größeren Durchmesser aufweisen, als die Heizungsrohrführung (1). Dies ist insbesondere bei großem Abstand der Heizungsrohre von der Wand vorteilhaft, um größere Stabilität gegenüber Biegekräften zu erzielen.

Dieser Zielsetzung dient auch die weitere in Fig. 4 dargestellte kegelstumpfförmige Ausführung der Abstandshalter (2). Diese Ausführung bietet sich insbesondere bei großen benötigten Stückzahlen an, bei denen eine Spritzgussfertigung der Abstandshalter wirtschaftlich wird.

In einer weiteren, in Fig. 5 dargestellten Ausführungsform sind die Abstandshalter (2) direkt mittels Befestigungselementen (3) an der Behälterwand (4) und die Heizungsrohrführung (1) mittels eines Rastelements (21) an den Abstandshaltern (2) befestigt. Bevorzugt greifen die Rastelemente (21) dabei in entsprechende Aufnahmen der Abstandshalter (2) ein und legen die Heizungsrohrführung (1) fest.

In Fig. 6 ist eine weitere erfindungsgemäße Ausführungsform dargestellt, bei der der Abstandshalter (2) so als Spritzguss-Formteil ausgebildet ist, dass die Aufnahme der Heizungsrohrführungen (1) und der Befestigungselemente (3) direkt angeformt ist und keine weiteren Konfektionsschritte erfordert.

Fig. 7 zeigt ein Fixierelement, mit dem eine Bewegung des Heizungsrohres (5) in der Heizungsrohrführung (1) verhindert werden kann. Dieses Fixierelement ist als Ring (7) ausgeführt, der zwischen Heizungsrohr (5) und Heizungsrohrführung (1) geklemmt wird und Heizungsrohr (5) und Heizungsrohrführung (1) kraftschlüssig gegeneinander verspannt.

Die vorgenannten Ausführungsformen erfordern ein Einfädeln der Heizungsrohre in die Heizungsrohrführungen und das Durchziehen der Heizungsrohre. Dies ist insbesondere dann vorteilhaft, wenn die Behälterwand eingerüstet ist.

In Einzelfällen kann es jedoch auch vorteilhaft sein, wenn die Heizungsrohre in die Heizungsrohrhalterungen eingelegt werden können.

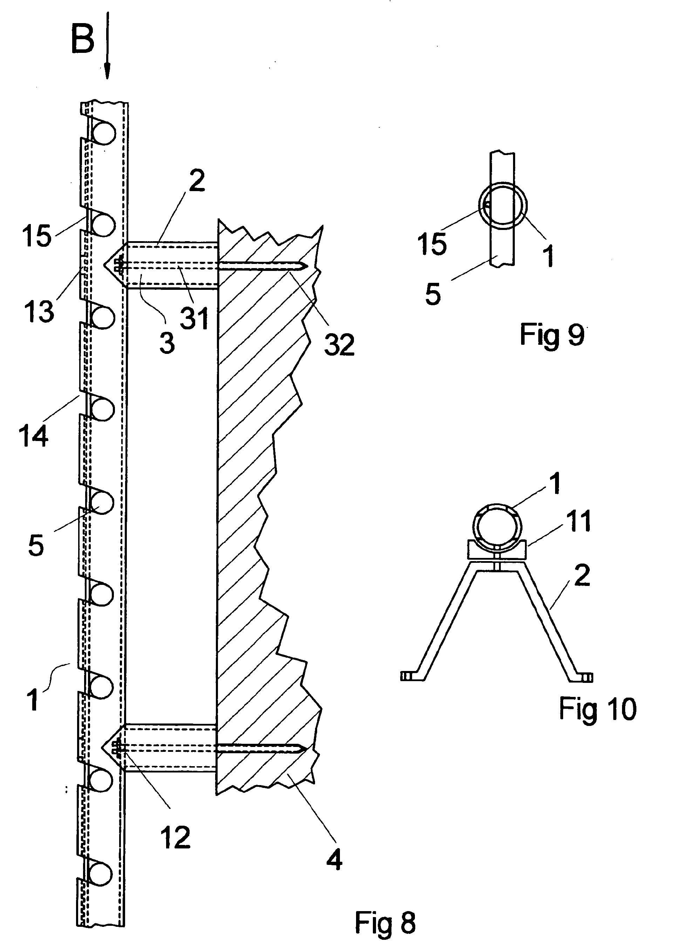

In Fig. 8 ist eine weitere abgewandelte Ausführungsform der Heizungsrohrhalterung (1) und in Fig. 9 dessen Ansicht B gezeigt, die dieses Einlegen der Heizungsrohre (5) in Öffnungen (14) ermöglicht. Die Öffnungen (14) sind vorzugsweise so ausgeführt, dass die Heizungsrohre (5) durch die Schwerkraft in ihrer vorgesehenen Position gehalten werden. Um ein Herausziehen der Heizungsrohre (5) aus den Heizungsrohrhalterungen (1) im Betrieb zu erschweren, können die Öffnungen (14) an ihrem freien Ende verjüngt ausgeführt sein, indem dort der lichte Abstand etwas kleiner ist als der Durchmesser der Heizungsrohre (5). Bevorzugt wird das Herausziehen der Heizungsrohre (5) aus der Heizungsrohrhalterung (1) dadurch verhindert, dass ein ein- oder mehrstückig ausgeführtes Sperrelement (15) in der Heizungsrohrhalterung (1) zwischen den Heizungsrohren (5) und dem freien Ende der Öffnungen (14) angeordnet ist.

In Fig_{.} 10 ist eine nicht zur Erfindung gehörende Heizungsrohrhalterung (1) gezeigt. Hierbei ist der Abstandshalter (2) beispielsweise als Spritzguss-Formteil ausgebildet, er weist in etwa eine Y-Form auf. Die Heizungsrohrhalterung (1) ist mittels einer Schiene 11 am Abstandshalter (2) befestigt.

## Patentansprüche

1. Halterung für Heizungsrohre in Anlagen zur Biogaserzeugung, bestehend wenigstens aus einer Heizungsrohrführung (1), einem Abstandshalter (2) und einem Befestigungselement (3), wobei die Heizungsrohrführung (1) und der Abstandshalter (2) überwiegend aus organischem Material bestehen, wobei die Heizungsrohrführung (1) mittels des vorzugsweise senkrecht dazu angeordneten Abstandshalters (2) von einer Behälterwand (4) beabstandet angeordnet ist, **dadurch gekennzeichnet, dass** die Heizungsrohrführung (1) und der Abstandshalter (2) Holz enthalten, und dass der Abstandshalter (2) als Rohr ausgeführt ist.

2. Halterung nach Anspruch 1, **dadurch gekennzeichnet, dass** die Heizungsrohrführung (1) und der Abstandshalter (2) einen rohrförmigen, insbesondere einen runden, elliptischen oder polygonalen Querschnitt aufweisen.

3. Halterung nach einem der vorangehenden Ansprüche, **dadurch gekennzeichnet, dass** die Heizungsrohrführung (1) und / oder der Abstandshalter (2) vernetztes oder unvernetztes thermoplastisches Polymermaterial oder duroplastisches Polymermaterial enthalten.

4. Halterung nach einem der vorangehenden Ansprüche, **dadurch gekennzeichnet, dass** die Heizungsrohrführung (1) und der Abstandshalter (2) mittels eines Befestigungselements (3) so an der Behälterwand angeordnet ist, dass der Abstandshalter (2) zwischen der Heizungsrohrführung (1) und der Behälternrand (4) verspannt ist.

5. Halterung nach einem der vorangehenden Ansprüche, **dadurch gekennzeichnet, dass** der Abstandshalter (2) an der Behälterwand befestigt und die Heizungsrohrführung (1) mittels eines Rastelements (21) an dem Abstandshalter (2) befestigt ist.

6. Halterung nach einem der vorangehenden Ansprüche, **dadurch gekennzeichnet, dass** der Abstandshalter (2) als Spritzgussformteil so ausgeführt ist, dass eine formschlüssige Aufnahme der Heizungsrohrführung (1) angeformt ist.

7. Halterung nach einem der vorangehenden Ansprüche, **dadurch gekennzeichnet, dass** das Befestigungselement (3) aus einer Gewindestange (33) besteht, die in der Behälterwand (4) verankert ist.

8. Halterung nach einem der vorangehenden Ansprüche, **dadurch gekennzeichnet, dass** der Abstandshalter (2) einen zur Behälterwand (4) hin zunehmenden Querschnitt aufweist.

9. Halterung nach einem der vorangehenden Ansprüche, **dadurch gekennzeichnet, dass** die Heizungsrohrführung (1) und oder die Abstandshalter (2) mehrteilig ausgeführt sind.

10. Halterung nach einem der vorangehenden Ansprüche, **dadurch gekennzeichnet, dass** der Abstandshalter (2) einen metallischen Werkstoff, insbesondere Edelstahl, aufweist.

11. Vorrichtung zur Heizung insbesondere in Anlagen zur Biogaserzeugung mit einer Halterung für Heizungsrohre gemäß einem der Ansprüche 1 bis 10.

## Claims

1. Holder for heating pipes in plants for biogas generation, consisting at least of a heating pipe guide (1), a spacer (2) and a fastening element (3), wherein the heating pipe guide (1) and the spacer (2) consist predominantly of organic material, wherein the heating pipe guide (1) is arranged at a distance from a tank wall (4) by means of the spacer (2) arranged preferably perpendicularly to the guide, **characterized in that** the heating pipe guide (1) and the spacer (2) comprise wood, and **in that** the spacer (2) is designed as a tube.

2. Holder according to Claim 1, **characterized in that** the heating pipe guide (1) and the spacer (2) have a tubular, in particular a round, elliptical or polygonal, cross section.

3. Holder according to either of the preceding claims, **characterized in that** the heating pipe guide (1) and/or the spacer (2) comprise crosslinked or uncrosslinked thermoplastic polymer material or duroplastic polymer material.

4. Holder according to one of the preceding claims, **characterized in that** the heating pipe guide (1) and the spacer (2) are arranged on the tank wall by means of a fastening element (3) in such a way that the spacer (2) is braced between the heating pipe guide (1) and the tank wall (4).

5. Holder according to one of the preceding claims, **characterized in that** the spacer (2) is fastened to the tank wall and the heating pipe guide (1) is fastened to the spacer (2) by means of a latching element (21).

6. Holder according to one of the preceding claims, **characterized in that** the spacer (2) is configured as an injection moulding in such a way that a positively locking receptacle for the heating pipe guide (1) is integrally formed.

7. Holder according to one of the preceding claims, **characterized in that** the fastening element (3) consists of a threaded rod (33) which is anchored in the tank wall (4).

8. Holder according to one of the preceding claims, **characterized in that** the spacer (2) has a cross section which increases towards the tank wall (4).

9. Holder according to one of the preceding claims, **characterized in that** the heating pipe guide (1) and/or the spacers (2) have a multi-part design.

10. Holder according to one of the preceding claims, **characterized in that** the spacer (2) comprises a metallic material, in particular stainless steel.

11. Apparatus for heating, in particular in plants for biogas generation, comprising a holder for heating pipes according to one of Claims 1 to 10.

## Revendications

1. Support pour tubes chauffants dans des installations de production de biogaz, constitué d'au moins un guidage de tube chauffant (1), d'un élément d'espacement (2) et d'un élément de fixation (3), le guidage de tube chauffant (1) et l'élément d'espacement (2) se composant principalement de matériau organique, le guidage de tube chauffant (1) étant disposé à distance d'une paroi de récipient (4) au moyen de l'élément d'espacement (2) disposé de préférence perpendiculairement à celui-ci, **caractérisé en ce que** le guidage de tube chauffant (1) et l'élément d'espacement (2) contiennent du bois, et **en ce que** l'élément d'espacement (2) est réalisé sous forme de tube.

2. Support selon la revendication 1, **caractérisé en ce que** le guidage de tube chauffant (1) et l'élément d'espacement (2) présentent une section transversale de forme tubulaire, notamment ronde, elliptique ou polygonale.

3. Support selon l'une quelconque des revendications précédentes, **caractérisé en ce que** le guidage de tube chauffant (1) et/ou l'élément d'espacement (2) contiennent un matériau polymère thermoplastique réticulé ou non réticulé ou un matériau polymère duroplastique.

4. Support selon l'une quelconque des revendications précédentes, **caractérisé en ce que** le guidage de tube chauffant (1) et l'élément d'espacement (2) sont disposés au moyen d'un élément de fixation (3) sur la paroi du récipient de telle sorte que l'élément d'espacement (2) soit serré entre le guidage de tube chauffant (1) et la paroi du récipient (4).

5. Support selon l'une quelconque des revendications précédentes, **caractérisé en ce que** l'élément d'espacement (2) est fixé sur la paroi du récipient et le guidage de tube chauffant (1) est fixé au moyen d'un élément d'encliquetage (21) sur l'élément d'espacement (2).

6. Support selon l'une quelconque des revendications précédentes, **caractérisé en ce que** l'élément d'espacement (2) est réalisé sous forme de pièce moulée par injection de telle sorte qu'un logement du guidage de tube chauffant (1) soit façonné par engagement par coopération de forme.

7. Support selon l'une quelconque des revendications précédentes, **caractérisé en ce que** l'élément de fixation (3) se compose d'une tige filetée (33) qui est ancrée dans la paroi du récipient (4).

8. Support selon l'une quelconque des revendications précédentes, **caractérisé en ce que** l'élément d'espacement (2) présente une section transversale augmentant vers la paroi du récipient (4).

9. Support selon l'une quelconque des revendications précédentes, **caractérisé en ce que** le guidage de tube chauffant (1) et/ou les éléments d'espacement (2) sont réalisés en plusieurs parties.

10. Support selon l'une quelconque des revendications précédentes, **caractérisé en ce que** l'élément d'espacement (2) présente un matériau métallique, notamment de l'acier spécial.

11. Dispositif de chauffage, en particulier dans des installations de production de biogaz, avec un support pour tubes chauffants selon l'une quelconque des revendications 1 à 10.
